# EUROPEAN PATENT SPECIFICATION

(11) **EP 2 387 398 B1**
(45) Date of publication and mention of the grant of the patent: **07.10.2015**
(21) Application number: 10701937.4
(22) Date of filing: 18.01.2010
(51) Int. Cl.: A61K 31/352, A61K 31/4196, A61K 31/7048, A61K 45/06, A61P 31/10

(54) **FULVIC ACID IN COMBINATION WITH FLUCONAZOLE OR AMPHOTERICIN B FOR THE TREATMENT OF FUNGAL INFECTIONS**
FULVINSÄURE IN KOMBINATION MIT FLUCONAZOL ODER AMPHOTERICIN B ZUR BEHANDLUNG VON PILZINFEKTIONEN
ACIDE FULVIQUE ASSOCIE A DU FLUCONAZOLE OU DE L'AMPHOTERICINE B DANS LE TRAITEMENT D'INFECTIONS FONGIQUES

(30) Priority: 19.01.2009 GB 0900786
(43) Date of publication of application: 23.11.2011
(73) Proprietor: Natracine UK Limited, Macclesfield, Cheshire SK10 4QH (GB); Donald, Heather June, 0001 Pretoria (ZA)
(72) Inventor: WARN, Peter, Worsley Manchester M28 144 (GB); LEIVERS, Stephen William, Wymondham Norfolk NR18 OEA (GB)
(74) Representative: Kremer, Simon Mark
(86) International application number: PCT/IB2010/050213
(87) International publication number: WO 2010/082182

(56) References cited:
- EP-A1- 0 955 056
- WO-A1-00/19999
- WO-A2-2007/125492
- WO-A2-2009/140215

## Description

### BACKGROUND OF THE INVENTION

This invention relates to fulvic acid in combination with one or more antifungal compounds for use in the therapeutic treatment or prophylaxis of various conditions in the human or animal body.

Fulvic acid is one of the substances formed during the decay of organic matter in the environment. It is soluble in water under all pH conditions and is generally of a lower molecular size and weight and lower in colour intensity than the humic acids also produced during the decay process.

Although fulvic acid occurs naturally in low levels in soil and water, it is difficult to isolate. A known process for yielding fulvic acid for use in medicinal applications is by a controlled wet oxidation of bituminous coal as described in US Patent No. 4,912,256. The use of such fulvic acid for treatment of inflammation, acne, eczema, bacterial, fungal and viral infections has been previously disclosed in International Patent Publication WO00/19999. In addition, US Patent Nos. 4,999,202 and 5,204,368 disclose compositions containing fulvic acid, salt or a derivative thereof, which have bacteriostatic or bacteriocidal properties and are useful as disinfectants.

Coal derived fulvic acids contain high concentrations of heavy metals such as aluminium, mercury, cadmium, chromium and lead that must be avoided in pharmaceutical preparations. A fulvic acid composition derived from a carbohydrate source (CHD-FA) by wet oxidation has been previously disclosed in International Patent Publication WO2007/125492. This CHD-FA is particularly useful for pharmaceutical application in that is has a low content of heavy metals. WO0019999 relates to pharmaceutical compositions comprising a fulvic acid, salt, ester or derivative thereof as active ingredients. The compositions are preferably administered orally or topically for treating a condition in a human or an animal. The condition may, for example, be inflammation, acne, eczema or bacterial or fungal or viral infections.

WO2007125492 relates to a composition which comprises an acidic component, generally fulvic acid, having a molecular weight not exceeding 20,000 Daltons and a low content of the elements aluminium, mercury, cadmium, chromium and lead. The acidic component is preferably carbohydrate derived and preferably using a wet oxidation process.

EP0955056 relates to a colony stimulating factor (CSF-1) which is a lymphokine reported to be useful in treating or preventing bacterial, viral or fungal infections, neoplasms, leukopenia, wounds, and in overcoming the immunosuppression induced by chemotherapy or resulting from other causes. CSF-1 is reported as being obtained in usable amounts by recombinant methods, including by cloning and expression of the murine and human DNA sequences encoding this protein.

### SUMMARY OF THE INVENTION

According to embodiments of the invention there are provided the following compositions:
A composition comprising a combination of:
   a) a minimum concentration of 0.5% (w/v) carbohydrate derived fulvic acid (CHD-FA) or a salt thereof, and
   b) fluconazole,
   for use in a method of treatment of a fungal infection of a human or non-human animal body, the method comprising administering to a subject in need thereof a therapeutically effective amount of the composition, wherein the fungal infection is caused by a drug-resistant fungus, and wherein the fungal infection is a Candida albicans, Candida tropicalis, Candida parapsilosis or Candida krusei fungal infection.
A composition comprising a combination of:
   a) a minimum concentration of 1% (w/v) CHD-FA, and
   b) fluconazole,
   for use in a method of treatment of a fungal infection of a human or non-human animal body, the method comprising administering to a subject in need thereof a therapeutically effective amount of the composition, wherein the fungal infection is caused by a drug-resistant fungus, and wherein the fungal infection is a Candida glabrata fungal infection.
A composition comprising a combination of:
   a) a minimum concentration of 0.25% (w/v) CHD-FA, and
   b) amphotericin B,
   for use in a method of treatment of a fungal infection of a human or non-human animal body, the method comprising administering to a subject in need thereof a therapeutically effective amount of the composition, wherein the fungal infection is caused by a drug-resistant fungus, and wherein the fungal infection is an Aspergillus fungal infection.

The fulvic acid, or salt thereof can have any pH, from acid to basic. For example, the pH of the fulvic acid can be raised by converting the acid into a salt, such as the sodium or potassium salt. This may be achieved by adding a suitable hydroxide to the fulvic acid.

The fulvic acid is carbohydrate derived (CHD-FA) preferably produced by the method described in WO 2007/125492. In particular, the CHD-FA may be derived from a saccharide. Typically, the CHD-FA has a molecular weight not exceeding 20,000 Daltons and a low content of the elements aluminium, mercury, cadmium, and chromium. The CHD-FA is manufactured by subjecting a carbohydrate to wet oxidation and then treating the reaction product obtained to remove substantially all acidic components with a molecular weight exceeding 20,000 Daltons.

Fluconazole is a known antifungal agent and is described as, for example, Item 4122 of the Merck Index, 14th Ed. The fluconazole can be administered in the form of an ester and it is to be understood that the term "fluconazole" as used herein and in the claims includes esters and other suitable pharmaceutical forms of fluconazole.

Amphotericin B is also a known antifungal agent and is described as, for example, Item 585 of the Merck Index, 14th Ed.

The combination of fulvic acid and fluconazole has surprisingly been found to be effective when administered against fluconazole-resistant fungi. In particular, the combination of fulvic acid and fluconazole is effective when administered against fluconazole-resistant *Candida spp.*

Amphotericin B has surprisingly been found to be effective at a lower, non-toxic dose when administered against fungal species in combination with fulvic acid.

In one form of the invention the combination comprises from about 10 ml/kg of a solution of about 0.25% to about 1% fulvic acid or a salt, ester or derivative thereof and about 10mg/kg of fluconazole.

The amphotericin B may be present in the combination at an effective dose that is non-toxic to the subject. More particularly, the combination comprises about 0.25% fulvic acid and from about 0.06mg/l to about 0.5mg/l of amphotericin B.

Also described herein is a pharmaceutical composition comprising the combination as described above as the active ingredients.

The pharmaceutical composition may be in a form suitable for oral administration, or topical administration or any other suitable form of administration. For example, the pharmaceutical composition may be formulated into a liquid, tablet, capsule or the like or into a cream, or ointment.

The human or animal to be treated may be immunosuppressed or immunocompromised.

The diseases or condition treated is caused by a drug-resistant fungus.

The diseases or condition treated is caused by *Candida spp,* or by *Aspergillus spp*.

Also described herein is the use of the combination described above in the manufacture of a pharmaceutical composition for treatment or prophylaxis of a disease or condition of the human or animal body.

### BRIEF DESCRIPTION OF THE DRAWINGS

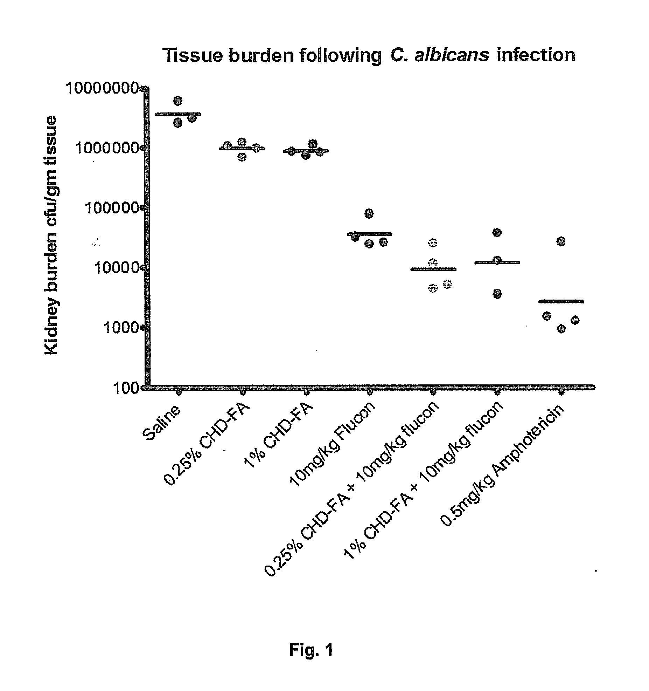

- **Figure 1**: shows kidney tissue burden following *Candida albicans* infection in mice treated with different concentrations of CHD-FA alone or in combination with fluconazole.

### DETAILED DESCRIPTION OF THE INVENTION

Drug resistance is a major problem for the treatment of diseases and conditions caused by fungal agents, such as has occurred with the use of fluconazole for the treatment of *Candida* infection and the use of amphotericin B in the treatment of *Aspergillus.* In particular, use of amphotericin B in the treatment of *Aspergillus* infections is no longer effective, as the dose required to inhibit *Aspergillus* is toxic to the subject (3mg/l).

In addition, the opportunistic fungal infections that occur in immunocompromised, or immunosuppressed patients by are difficult to control with antifungal agents. A treatment strategy is therefore needed in these patients, particularly cancer patients who are receiving anti-cancer drugs and other patients on any drugs that cause immunosuppression.

Three studies were conducted to evaluate the antifungal characteristics of fulvic acid against specific organisms. The fulvic acid used in these studies was that described in, and produced by the method described in WO 2007/125492 and is hereinafter referred to as CHD-FA. In brief, the fulvic acid was derived from a carbohydrate, in particular a saccharide. The CHD-FA has a molecular weight not exceeding 20, 000 Daltons and a low content, i.e. less than 30 ppm, of elements such as aluminium, mercury, cadmium, chromium, lead, silver, arsenic and beryllium. The CHD-FA was manufactured by subjecting the carbohydrate to wet oxidation and then treating the reaction product obtained to remove substantially all acidic components with a molecular weight exceeding 20, 000 Daltons.

In the first study, kidney burdens of *Candida albicans* was determined as an indication of the efficacy of increasing concentrations of CHD-FA alone or in combination with the antifungal compound, fluconazole. Results showed that fulvic acid significantly enhances the activity of fluconazole against *Candida albicans.*

In the second study, the efficacy of fulvic acid alone or in combination with the antifungal compound amphotericin B against *Aspergillus* or zygomycetes was determined by quantitative colony counts in tissue culture plates.

In the third study, the efficacy of fluconazole in combination with fulvic acid against drug resistant strains of *Candida spp* was determined by quantitative colony counts in tissue culture plates.

All solutions are set out as percentages of weight per volume.

The following examples are for the purpose of illustration only and are not to be construed as limiting on the invention in any way.

### EXAMPLE 1 - In vivo efficacy of CHD-FA against Candida

### 1.1. PHYSICAL PROPERTIES OF NEW ANTIBIOTIC RESISTANCE MODULATING AGENT ALSO KNOWN AS CARBOHYDRATE DERIVED FULVIC ACID (CHD-FA)

CHD-FA was reconstituted as a 4% solution. The solution was stored at room temperature in the dark since delivery. The 4% CHD-FA solution was a yellow/brown slightly viscous solution with a strong odour and a pH of 2.1 at 25°C.

### 1.2. METHODS

### 1.2.1 Regulatory Issues

All animal experiments were performed under UK Home Office Licence 40/3101 Invasive Fungal Diseases (Licence Holder Dr Peter Warn) and with local ethical committee clearance. All experiments will be performed by technicians that have completed parts 1, 2 and 3 of the Home Office Personal License course and hold a current personal license. All experiments were performed in dedicated Biohazard 2 facilities within the Biological Services Unit of The University of Manchester (this site holds a Certificate of Designation).

### 1.2.2 Animal Model

Mice used in this study, male CD1 mice (an outbred strain that is very similar to Swiss mice) were supplied by Charles River (Margate UK) and were specific pathogen free (16-18g at delivery). All mice weighed 20-22g at the time of immunosuppression.

Mice were housed in individual ventilated cages (IVCs) that are supplied with HEPA filtered air. Sterile aspen chip bedding was supplied in pre-autoclaved boxes. Sterile water was provided ad libitum using disposable pouches. Standard mouse chow was provided ad libitum (food was moistened into mash if mice demonstrated signs of sepsis).

Mice experienced a 12hour light dark cycle at 22±1°C, 55-60% relative humidity and background noise of <60db.

Animals were treated using either 30G disposable 'insulin' Monojects (for iv or ip administrations) or reusable 19G gavage needles.

All animals were immunosuppressed with a single dose of 200mg/kg cyclophosphamide (Pharmacia) intraperitoneally (ip) 3 days before infection. This results in a profound state of neutropenia lasting for 3-4 days post infection.

### 1.2.3 Experiment Duration

The experiment was continued till 53 hours post infection.

### 1.2.4 Animal Group Size

For the combination study animals were treated in groups of 4 mice per treatment group.

### 1.2.5 Infection

Mice were infected with 0.2ml of a suspension of FA7070 in PBS + 0.05% tween 80 containing 1.5 x 10⁵ blastoconidia/ml i.e. 3.0 x 10⁴ yeasts per mouse. Following infection all mice were observed at least 4 times daily. Animals exceeding the severity band of the experiment were humanely euthanized.

### 1.2.6 Antifungal Treatment

Mice were treated 5 hours post infection with either:
a) 0.125ml of 2% CHD-FA administered by gavage (assuming mice are 25g at the time of treatment). CHD-FA was administered twice daily (total of 6 doses administered).
b) 0.125ml of 0.5% CHD-FA administered by gavage (assuming mice are 25g at the time of treatment). CHD-FA was administered twice daily (total of 6 doses administered).
c) 10mg/kg fluconazole administered intravenously in 5% glucose (in 0.25ml)
d) Combination therapy of 0.125ml of 2% CHD-FA administered twice daily orally and 10mg/kg fluconazole administered intravenously in 5% glucose.
e) Combination therapy of 0.125ml of 0.5% CHD-FA administered twice daily orally and 10mg/kg fluconazole administered intravenously in 5% glucose.
f) 0.5mg/kg amphotericin B (diluted in 5% glucose) administered intraperitoneally.
g) Vehicle treated mice will be given 0.125ml of 0.9% saline by gavage administered twice daily and 0.25ml 5% glucose administered intravenously.

### 1.2.7 End of Animal Experiment

53 hours post infection all animals were euthanized using a schedule 1 procedure. All animals were weighed; kidneys were removed immediately and homogenized in ice cold sterile phosphate buffered saline. Kidney homogenates were quantitatively cultured onto Sabouraud Dextrose agar and incubated at 37°C for up to 4 days and colonies counted.

### 1.2.8 Statistical Analysis

The data from the culture burdens was analyzed by the Kruskal-Wallis test using Stats Direct.

### 1.3 RESULTS

### 1.3.1 Kidney burdens

A summary of the kidney burdens is detailed in Figure 1.

In this study there were no side effects noted after treatment with CHD-FA and study was ended at 53 hours post infection due to severe infection in the vehicle treated group.

### 1.3.2. Statistical Analysis

**Table 1: Kruskal-Wallis: all pairwise comparisons (Conover-Inman)**

| | Saline | 0.25% CHD-FA | 1% CHD-FA | 10mg/kg Fluconazole | 0.25% CHD-FA + 10mg/kg fluconazole | 1% CHD-FA + 10mg/kg fluconazole | 0.5mg/kg Amphotericin |
|---|---|---|---|---|---|---|---|
| Saline | | NS (0.06) | 0.028 | <0.0001 | <0.0001 | <0.0001 | <0.0001 |
| 0.25% CHD-FA | | | NS (0.67) | 0.0026 | <0.0001 | <0.0002 | <0.0001 |
| 1 % CHD-FA | | | | 0.007 | <0.0001 | 0.006 | <0.0001 |
| 10mg/kg Fluconazole | | | | | 0.044 | NS (0.19) | 0.004 |
| 0.25% CHD-FA + 10mg/kg fluconazole | | | | | | NS (0.51) | NS (0.29) |
| 1% CHD-FA + 10mg/kg fluconazole | | | | | | | NS (0.11) |
| 0.5mg/kg Amphotericin | | | | | | | |

| | | | | | | | |
|---|---|---|---|---|---|---|---|
| **NS** = **not significant** | | | | | | | |

### 1.4 SUMMARY

- Experiments were established using 0.125ml gavages of 2% and 0.5% CHD-FA twice daily (equivalent to 1% and 0.25% CHD-FA administered at 10ml/kg).
- 5ml/kg of 2% or 0.5% CHD-FA (equivalent to 10ml/kg at 1% and 0.25% CHD-FA) was well tolerated in mice.
- 5ml/kg of 2% or 0.5% CHD-FA (equivalent to 10ml/kg at 1% and 0.25% CHD-FA) was effective at reducing the kidney burden on mice infected with *Candida albicans.* The burden following treatment was significantly lower than vehicle treated mice (∼0.6 log₁₀ cfu/gram reduction)
- 5ml/kg of 2% or 0.5% CHD-FA (equivalent to 10ml/kg at 1% and 0.25% CHD-FA) was additive when used in combination with fluconazole. The combined reduction in tissue burden was significantly superior to either treatment used as monotherapy.

### EXAMPLE 2 - In vitro efficacy of CHD-FA against Aspergillus and Zygomycetes

### 2.1 PHYSICAL PROPERTIES OF CHD-FA ALSO KNOWN AS FULVIC ACID

CHD-FA was reconstituted as a 4% solution. The solutions were stored at room temperature in the dark since delivery. The 4% CHD-FA solution was a yellow/brown slightly viscous solution with a strong odour and a pH of 2.1 at 25°C.

### 2.2 METHODS - Preliminary Experiment

### 2.2.1 Fungal Isolates

Susceptibility tests were performed on the following isolates which are all strains isolated from cases of human clinical disease.
(i) 2 x *Aspergillus fumigatus.*
(ii) 2 x *Aspergillus terreus -* these strains are moderately resistant *in vitro* and resistant *in vivo* to amphotericin B which is typical of *A. terreus* strains.
(iii) 2 x *Aspergillus flavus -* these strains are of intermediate susceptibility *in vitro* and respond poorly *in vivo* to amphotericin B (1 strain).
(iv) 2 x *Aspergillus flavus -* these strains are of intermediate susceptibility *in vitro* and respond poorly *in vivo* to amphotericin B (1 strain).
(v) 2 x *Absidia corymbifera* - these strains are susceptible *in vitro* but respond very poorly *in vivo* to amphotericin B.
(vi) 2 x *Fusarium solani -* these strains are highly resistant *in vitro* and do not respond *in vivo* to amphotericin B.

Each culture was grown on Sabouraud agar at 37°C for 10 days to ensure purity and to allow spores to mature

### 2.2.2 Media

RPMI-1640 (Sigma, Dorset, UK) supplemented to 2% glucose (Sigma), buffered with morpholinopropanesulfonic acid (MOPS), (Sigma) and adjusted to pH 7.0 was used as recommended in the Clinical Laboratory Standards M38A document (Reference method for broth dilution antifungal susceptibility testing of conidium-forming filamentous fungi. Approved standard. Document M38-A 2002a. NCCLS, Wayne, PA. 2002. NCCLS, Wayne, PA, USA).

### 2.2.3 Preparation of the Inoculum

a) All fungi were cultured in ambient air at 35-37 °C on recovery medium (Sabouraud dextrose agar) for 8-10 days before testing.
b) Inoculum suspensions were prepared from day 8 to 10 day cultures grown on Sabouraud dextrose agar at 37°C in vented tissue culture flasks to avoid crosscontamination. Spores were harvested by flooding the surface of the growth with 25ml of sterile phosphate buffered saline plus 0.05% Tween 80. The spore count was adjusted using a counting chamber.
c) The inoculum was completely suspended by vigorous shaking on a vortex mixer for 15s. The final spore density in the MIC tests was between 0.5 x 104 and 5 x 104 CFU/mL as demonstrated by quantitative colony counts. Drug-free and cell-free controls were included. (The RPMI medium used in the plates was prepared at either 2 x or 4 x the final strength to allow for dilution once the inoculum and diluted CHD-FA was added).

### 2.2.4 Assay Conditions

Sterile plastic, disposable, microtitration plates with 96 flat-bottom wells were used.

**STEP 1** Addition of amphotericin B (stock solution prepared in 100% DMSO)
a) Column 1 of the microtitration tray was filled with 100µL of sterile water containing four times the final drug concentration (16mg/L amphotericin B).
b) Columns 2-12 were filled with 50µL of distilled water
c) 50µL amounts were taken from wells in column 1 and diluted two-fold by transferring them to column 2 with a multichannel pipette (± 2% coefficient of variation). 50 µL samples were then removed from column 2 and transferred to column 3, and so on through to column 10. The last 50 µL of diluted drug is then discarded. Thus, each well in columns 1-10 will contain 50µL of water containing four times the final antifungal drug concentrations.

### STEP 2 Addition of CHD-FA

Stock solutions of CHD-FA were produced containing 4%, 2%, 1%, 0.5% and 0.25% of the native compound.

100µL of the diluted CHD-FA was added to microtitration trays in so that row A contains a final dilution of 2% row B, 1%, row C 0.5%, row D 0.25% row E 0.125% and row F diluent only.

### STEP 3 Addition of Aspergillus or Zygomycetes strains

50µL of the diluted spore suspension in 4 x RPMI was added to all wells. This produces a well containing 200 µL final volume (made up of 50µL diluted antifungal, 100µL diluted CHD-FA or diluent, 50µL of 4 X RPMI containing fungal spores)

### STEP 4 Incubation of Plates

All plates were incubated at 37°C in air in a darkened incubator.

### STEP 5 Reading of Plates

Plates were read visually with the endpoint taken as the lowest concentration of drug that inhibited growth by 50% of that of the drug free control.

### 2.3. RESULTS - Preliminary Experiment

### 2.3.1 MICs against CHD-FA and Amphotericin B

MICs against the single agents demonstrated that CHD-FA at 4%, 2% and 1% inhibited the growth of *Aspergillus* and Zygomycetes for at least 24 hours. The MIC values for CHD-FA and Amphotericin B are detailed in Table 2.

**Table 2: Efficacy of CHD-FA against Aspergillus and zygomycetes**

| **Isolate Species** | **Isolate Number** | **MIC (%) CHD-FA** | **Amphotericin MIC (mg/L)** |
|---|---|---|---|
| *A. fumigatus* | 1 | 0.5 | 0.5 |
| *A. fumigatus* | 2 | 0.5 | 0.25 |
| *A. terreus* | 1 | 0.5 | 0.5 |
| *A. terreus* | 2 | 0.5 | 1.0 |
| *A. flavus* | 1 | 0.5 | 4.0 |
| *A. flavus* | 2 | 0.25 | 0.5 |
| *Absidia* | 1 | 0.25 | 0.25 |
| *Absidia* | 2 | 0.25 | 0.06 |
| *Fusarium* | 1 | 0.25 | 2.0 |
| *Fusarium* | 2 | 0.25 | 2.0 |

### 3.3.2 MICs against the Combination of CHD-FA and Amphotericin B

MICs against the combination of CHD-FA and amphotericin B are shown in table 3. It is of note than none of the combinations were antagonistic but the combination was highly effective against a strain of *Aspergillus fumigatus* that is resistant *in vitro* and *in vivo* to amphotericin B.

**Table 3: Efficacy of the combination of CHD-FA and amphotericin B against Aspergillus**

| **Isolate Species** | **CHD-FA Dilution** | **MIC CHD-FA** | **Amphotericin MIC** | **Combination MIC** | **Effect** |
|---|---|---|---|---|---|
| *A. fumigates* strain 1 | 0.5 | No Growth | No Growth | No Growth | possible synergy |
| | 0.25 | No Growth | No Growth | No Growth | |
| | 0.125 | Growth | 0.06 | <0.06 | |
| *A. fumigates* strain 2 | 0.5 | No Growth | No Growth | No Growth | neither synergy nor antagonism |
| | 0.25 | No Growth | No Growth | No Growth | |
| | 0.125 | Growth | 0.125 | 0.125 | |
| *A.* t*erreus* strain 1 | 0.5 | No Growth | No Growth | No Growth | neither synergy nor antagonism |
| | 0.25 | No Growth | No Growth | No Growth | |
| | 0.125 | Growth | 0.25 | 0.25 | |
| *A. terreus* strain 2 | 0.5 | No Growth | No Growth | No Growth | neither synergy nor antagonism |
| | 0.25 | No Growth | No Growth | No Growth | |
| | 0.125 | Growth | 0.125 | 0.125 | |
| *A. flavus* strain 1 | 0.5 | No Growth | No Growth | No Growth | combination reduced MIC from resistant to highly susceptible |
| | 0.25 | No Growth | No Growth | No Growth | |
| | 0.125 | Growth | >4.0 | <0.06 | |
| *A. flavus* strain 2 | 0.5 | No Growth | No Growth | No Growth | neither synergy nor antagonism |
| | 0.25 | No Growth | No Growth | No Growth | |
| | 0.125 | Growth | 0.25 | 0.5 | |

### 2.4. SUMMARY

- CHD-FA does not demonstrate antagonistic activity when used in combination with amphotericin B *Aspergillus spp.*
- The combination of 0.25% CHD-FA with amphotericin B (0.06 - 0.5mg/l) inhibits the growth of all *Aspergillus* isolates tested regardless of the amphotericin B MIC of the isolates.

### EXAMPLE 3 - In vitro efficacy of a combination of fluconazole and CHD-FA against Candida spp.

### 3.1 PHYSICAL PROPERTIES OF CHD-FA ALSO KNOWN AS FULVIC ACID

CHD-FA was reconstituted as a 4% solution. The solutions were stored at room temperature in the dark since delivery. The 4% CHD-FA solution was a yellow/brown slightly viscous solution with a strong odour and a pH of 2.1 at 25°C.

### 3.2 METHODS - Preliminary Experiment

### 3.2.1 Fungal Isolates

Susceptibility tests were performed on 5 isolates of *Candida albicans* all clinical isolates (all with reduced susceptibility to fluconazole). Each culture was grown on Sabouraud agar at 37°C for 48 h to ensure purity.

### 3.2.2 Media

RPMI-1640 (Sigma, Dorset, UK) supplemented to 2% glucose (Sigma), buffered with morpholinopropanesulfonic acid (MOPS), (Sigma) and adjusted to pH 7.0 was used as recommended in the European Committee on Antimicrobial Susceptibility Testing (E.Dis. 7.1) (Rodriguez-Tudela, J. L., F. Barchiesi, J. Bille, E. Chryssanthou, M. Cuenca-Estrella, D. Denning, J. P. Donnelly, B. Dupont, W. Fegeler, C. Moore, M. Richardson, P. E. Verweij, and the Subcommittee on Antifungal Susceptibility Testing (AFST) of the ESCMID European Committee for Antimicrobial Susceptibility Testing (EUCAST) 2003. Method for the determination of minimum inhibitory concentration (MIC) by broth dilution of fermentative yeasts. Clinical Microbiology and Infection 9:I-VIII)

### 3.2.3 Preparation of the Inoculum

a) All yeasts were cultured in ambient air at 35-37 °C on recovery medium (Sabouraud dextrose agar) for 18-24 h before testing.
b) The inoculum was prepared by picking five distinct colonies from 18 to 24 h cultures and suspending them in 5 mL of sterile distilled water.
c) The inoculum was completely suspended by vigorous shaking on a vortex mixer for 15s. The cell density was adjusted to the density of a 0.5 McFarland standard by adding sterile distilled water and measuring absorbance in a spectrophotometer at a wavelength of 530 nm. This gave a yeast suspension of 1-5 x 10⁶cfu/mL. A working suspension was prepared by a further dilution of 1 in 10 further in 4 X RPMI to give 1-5 x 10⁵cfu/mL (The RPMI medium used in the plates was prepared at 4 x the final strength to allow for a 75% dilution once the inoculum and diluted CHD-FA was added).

### 3.2.4 Assay Conditions

Sterile plastic, disposable, microtitration plates with 96 flat-bottom wells were used.

### STEP 1 Addition of fluconazole

a) Column 1 of the microtitration tray was filled with 100µL of sterile water containing four times the final drug concentration (512 mg/L fluconazole).
b) Columns 2-12 were filled with 50µL of distilled water
c) 50µL amounts were taken from wells in column 1 and diluted two-fold by transferring them to column 2 with a multichannel pipette (± 2% coefficient of variation). 50 µL samples were then removed from column 2 and transferred to column 3, and so on through to column 10. The last 50 µL of diluted drug is then discarded. Thus, each well in columns 1-10 will contain 50µL of water containing four times the final antifungal drug concentrations.

### STEP 2 Addition of CHD-FA

Stock solutions of CHD-FA were produced containing 4%, 2%, 1%, 0.5% and 0.25% of the native compound. 100µL of the diluted CHD-FA was added to microtitration trays in so that row A contains a final dilution of 2% row B, 1%, row C 0.5%, row D 0.25% row E 0.125% and row F diluent only.

### STEP 3 Addition of Candida albicans

50µL of the diluted *Candida albicans* suspension in 4 x RPMI was added to all cells. This produces a well containing 200 µL final volume (made up of 50µL diluted fluconazole, 100µL diluted CHD-FA or diluent, 50µL of 4 X RPMI containing *Candida albicans*)

### STEP 4 Incubation of Plates

All plates were incubated at 37oC in air in a darkened incubator.

### STEP 5 Reading of Plates

Plates were read visually with the endpoint taken as the lowest concentration of drug that inhibited growth by 50% of that of the drug free control.

### 3.3.1 RESULTS - Preliminary Experiment

Initial tests demonstrated that CHD-FA at 4%, 2% and 1% inhibited the growth of *Candida albicans* for at least 24 hours when combined with 1 X RPMI 1640 culture medium, growth in 0.5% CHD-FA occurs at similar levels to solvent controls. The inhibition of growth (4%, 2% and 1%) was possibly due to the strongly acidic pH. At the native pH of CHD-FA (pH 2.1) no synergy or antagonism with fluconazole could be detected.

The pH of CHD-FA was adjusted to pH7.0 using 10M NaOH solution (the ideal pH for fluconazole activity). This resulted in a brown slightly viscous solution with a strong characteristic odour. Susceptibility assays were repeated as above.

As previously noted with native CHD-FA, 4%, 2% and 1% solutions inhibited the growth of *Candida albicans* for at least 24 hours when combined with 1 X RPMI 1640 culture medium, growth in 0.5% CHD-FA occurs at similar levels to solvent controls. No synergy or antagonism with fluconazole could be detected.

The MICs of the *Candida albicans* strains with and without CHD-FA are listed in Table 4 (the pH of CHD-FA was adjusted to 7.0).

**Table 4: Minimum Inhibitory Concentrations of Fluconazole (mg/L) combined with CHD-FA**

| ***Candida* Strain** | **2% CHD-FA** | **1% CHD-FA** | **0.5% CHD-FA** | **0.25% CHD-FA** | **0.125% CHD-FA** | **Solvent** |
|---|---|---|---|---|---|---|
| 1 | No growth | No growth | 16 | 16 | 16 | 128 |
| 2 | No growth | No growth | 32 | 16 | 16 | 16 |
| 3 | No growth | No growth | 2 | 16 | 16 | 16 |
| 4 | No growth | No growth | 32 | 32 | 32 | 32 |
| 5 | No growth | No growth | 32 | 32 | 16 | 16 |

### 3.4 METHODS - Main Experiment

### 3.4.1 Fungal Isolates

Susceptibility tests were performed on 40 clinical isolates isolates of *Candida* of which 38 had reduced susceptibility to fluconazole. The group comprised 25 C. *albicans,* 11 C. *glabrata* (all with reduced susceptibility to fluconazole), and 4 C. *tropicalis* (all with reduced susceptibility to fluconazole). Each culture was grown on Sabouraud agar at 37°C for 48 h to ensure purity.

### 3.4.2 Media

As indicated in 3.2.2.

### 3.4.3 Preparation of the Inoculum

As indicated in 3.2.3.

### 3.4.4 Assay Conditions

Sterile plastic, disposable, microtitration plates with 96 flat-bottom wells were used.

### STEP 1 Addition of fluconazole

a) Column 1 of the microtitration tray was filled with 100µL of sterile water containing four times the final drug concentration (512 mg/L fluconazole).
b) Columns 2-12 were filled with 50µL of distilled water
c) 50µL amounts were taken from wells in column 1 and diluted two-fold by transferring them to column 2 with a multichannel pipette (± 2% coefficient of variation). 50 µL samples were then removed from column 2 and transferred to column 3, and so on through to column 10. The last 50 µL of diluted drug is then discarded. Thus, each well in columns 1-10 will contain 50µL of water containing four times the final antifungal drug concentrations.

### STEP 2 Addition of CHD-FA

Stock solutions of CHD-FA were produced containing 4% and 2% (also 1 % and 0.5% for *Candida parapsilosis* and *Candida krusei*)*.* 100µL of the diluted CHD-FA was added to microtitration trays in so that rows contain final dilutions of:
For *Candida albicans* and *tropicalis:* 1st row of pair 0.5% CHD-FA, 2nd row of pair solvent only
For *Candida glabrata:* 1st row of triplet 1% CHD-FA, 2nd row of triplet 0.5% CHD-FA, 3rd row of triplet solvent only
For *Candida parapsilosis* and *Candida krusei* rows included 2%, 1%, 0.5%, 0.25 and 0.125% CHD-FA and a row of solvent only.

All the above concentrations were mixed with fixed concentrations of fluconazole at 128, 32, 8, 2, 0.5mg/L and solvent for synergy/antagonism assessment.

### STEP 3 Addition of Candida spp

50µL of the diluted *Candida spp* suspension in 4 x RPMI was added to all wells. This produces a well containing 200 µL final volume (made up of 50µL diluted fluconazole, 100µL diluted CHD-FA or diluent, 50µL of 4 X RPMI containing *Candida)*

### STEP 4 Incubation of Plates

All plates were incubated at 37oC in air in a darkened incubator.

### STEP 5 Reading of Plates

Plates were read visually with the endpoint taken as the lowest concentration of drug that inhibited growth by 50% of that of the drug free control.

### 3.5. RESULTS - Main Experiment

Results are summarized in Tables 5 to 10.

No regrowth of *Candida* occurred on prolonged incubation (96 hours) at 37°C or room temperature.

**Table 5 Efficacy of CHD-FA (0.5%) in combination with fluconazole against Candida albicans**

| **Isolate Species** | **Isolate Number** | **Fluconazole MIC (mg/L) alone** | **Fluconazole MIC with 0.5% CHD-FA** |
|---|---|---|---|
| *C. albicans* | 1 | 128 | No Growth |
| *C. albicans* | 2 | 8 | No Growth |
| *C. albicans* | 3 | 16 | No Growth |
| *C. albicans* | 4 | 4 | No Growth |
| *C. albicans* | 5 | 128 | No Growth |
| *C. albicans* | 6 | >128 | No Growth |
| *C. albicans* | 7 | 128 | No Growth |
| *C*. *albicans* | 8 | 128 | No Growth |
| *C*. *albicans* | 9 | 64 | No Growth |
| *C*. *albicans* | 10 | 128 | No Growth |
| *C*. *albicans* | 11 | 0.5 | No Growth |
| *C*. *albicans* | 12 | 64 | No Growth |
| *C*. *albicans* | 13 | 0.5 | No Growth |
| *C*. *albicans* | 14 | 32 | No Growth |
| *C*. *albicans* | 15 | 2 | No Growth |
| *C*. *albicans* | 16 | 64 | No Growth |
| *C*. *albicans* | 17 | >128 | No Growth |
| *C*. *albicans* | 18 | >128 | No Growth |
| *C*. *albicans* | 19 | 0.5 | No Growth |
| *C*. *albicans* | 20 | >128 | No Growth |
| *C*. *albicans* | 21 | 4 | No Growth |
| *C*. *albicans* | 22 | 2 | No Growth |
| *C*. *albicans* | 23 | 1 | No Growth |
| *C. albicans* | 24 | 1 | No Growth |
| *C*. *albicans* | 25 | >128 | No Growth |

**Table 6 Efficacy of CHD-FA (0.5%) in combination with fluconazole against Candida glabrata**

| **Isolate Species** | **Isolate Number** | **Fluconazole MIC (mg/L) alone** | **Fluconazole MIC with 0.5% CHD-FA** |
|---|---|---|---|
| *C. glabrata* | 1 | >128 | >128 |
| *C*. *glabrata* | 2 | >128 | >128 |
| *C. glabrata* | 3 | >128 | >128 |
| *C. glabrata* | 4 | >128 | >128 |
| *C. glabrata* | 5 | >128 | Trace Growth |
| *C. glabrata* | 6 | >128 | >128 |
| *C. glabrata* | 7 | >128 | >128 |
| *C. glabrata* | 8 | >128 | >128 |
| *C. glabrata* | 9 | >128 | >128 |
| *C*. *glabrata* | 10 | >128 | >128 |
| *C*. *glabrata* | 11 | >128 | >128 |

**Table 7 Efficacy of CHD-FA (1%) in combination with fluconazole against Candida glabrata**

| **Isolate Species** | **Isolate Number** | **Fluconazole MIC (mg/L) alone** | **Fluconazole MIC with 0.5% CHD-FA** |
|---|---|---|---|
| *C. glabrata* | 1 | >128 | No Growth |
| *C. glabrata* | 2 | >128 | No Growth |
| *C. glabrata* | 3 | >128 | No Growth |
| *C. glabrata* | 4 | >128 | No Growth |
| *C*. *glabrata* | 5 | >128 | No Growth |
| *C**.** glabrata* | 6 | >128 | No Growth |
| *C. glabrata* | 7 | >128 | No Growth |
| *C. glabrata* | 8 | >128 | No Growth |
| *C. glabrata* | 9 | >128 | No Growth |
| *C. glabrata* | 10 | >128 | No Growth |
| *C. glabrata* | 11 | >128 | No Growth |

**Table 8 Efficacy of CHD-FA (0.5%) in combination with fluconazole against Candida tropicalis**

| **Isolate Species** | **Isolate Number** | **Fluconazole MIC (mg/L) alone** | **Fluconazole MIC with 0.5% CHD-FA** |
|---|---|---|---|
| *C. tropicalis* | 1 | 16 | No Growth |
| *C. tropicalis* | 2 | >64 | No Growth |
| *C*. *tropicalis* | 3 | 16 | No Growth |
| *C*. *tropicalis* | 4 | 16 | No Growth |

**Table 9 Efficacy of CHD-FA (0.5%) in combination with fluconazole against Candida parapsilosis**

| **Isolate Species** | **Isolate Number** | **Fluconazole MIC (mg/L) alone** | **Fluconazole MIC with 0.5% CHD-FA** | **CHD-FA (%) without fluconazole** |
|---|---|---|---|---|
| *C. parapsilosis* | 1 | 8 | No Growth | 0.25 |
| *C. parapsilosis* | 2 | 2 | No Growth | 0.5 |
| *C. parapsilosis* | 3 | 8 | No Growth | 0.5 |
| *C. parapsilosis* | 4 | 8 | No Growth | 0.5 |
| *C*. *parapsilosis* | 5 | 8 | No Growth | 0.5 |

**Table 10 Efficacy of CHD-FA (0.5%) in combination with fluconazole against Candida krusei**

| **Isolate Species** | **Isolate Number** | **Fluconazole MIC (mg/L) alone** | **Fluconazole MIC with 0.5% CHD-FA** | **CHD-FA (%) without fluconazole** |
|---|---|---|---|---|
| *C. krusei* | 1 | 32 | No Growth | 0.25 |
| *C. krusei* | 2 | 32 | No Growth | 0.5 |
| *C. krusei* | 3 | 32 | No Growth | 0.25 |
| *C. krusei* | 4 | 32 | No Growth | 0.5 |
| *C. krusei* | 5 | 32 | No Growth | 0.5 |

### 3.6 SUMMARY

- CHD-FA is equally effective as an antifungal agent *in vitro* whether examined at it's native pH of 2.1 or buffered to pH7.0.
- CHD-FA inhibits the growth of *Candida albicans, Candida tropicalis, Candida parapsilosis* and *Candida krusei* when used as a 0.5% *in vitro.*
- CHD-FA inhibits the growth of *Candida glabrata* when used as a 1% solution.
- The combination of 1% CHD-FA with fluconazole (0.25-128mg/l) inhibits the growth of all *Candida* isolates tested.
- CHD-FA is highly effective against strains of C. *krusei* that are intrinsically resistant to fluconazole.

## Claims

1. A composition comprising a combination of:
a) a minimum concentration of 0.5% (w/v) carbohydrate derived fulvic acid (CHD-FA) or a salt thereof, and
b) fluconazole,
for use in a method of treatment of a fungal infection of a human or non-human animal body, the method comprising administering to a subject in need thereof a therapeutically effective amount of the composition, wherein the fungal infection is caused by a drug-resistant fungus, and wherein the fungal infection is a *Candida albicans, Candida tropicalis, Candida parapsilosis* or *Candida krusei fungal* infection.

2. A composition comprising a combination of:
a) a minimum concentration of 1% (w/v) CHD-FA, and
b) fluconazole,
for use in a method of treatment of a fungal infection of a human or non-human animal body, the method comprising administering to a subject in need thereof a therapeutically effective amount of the composition, wherein the fungal infection is caused by a drug-resistant fungus, and wherein the fungal infection is a *Candida glabrata* fungal infection.

3. A composition comprising a combination of:
a) a minimum concentration of 0.25% (w/v) CHD-FA, and
b) amphotericin B,
for use in a method of treatment of a fungal infection of a human or non-human animal body, the method comprising administering to a subject in need thereof a therapeutically effective amount of the composition, wherein the fungal infection is caused by a drug-resistant fungus, and wherein the fungal infection is an *Aspergillus* fungal infection.

4. The composition for use according to any one of claims 1 to 3, wherein the salt comprises the sodium or potassium salt.

5. The composition for use according to claim 1 or claim 4 as dependent on claim 1, comprising about 10 ml/kg of a solution of about 0.25% to about 1% (w/v) of fulvic acid, or a salt thereof, and about 10mg/kg of fluconazole.

6. The composition for use according to claim 3 or claim 4 as dependent on claim 3, comprising a solution of about 0.25% (w/v) fulvic acid, or a salt thereof and from about 0.06mg/l to about 0.5mg/l of amphotericin B.

7. The composition for use according to any one of claims 1 to 6 which is administered in the form of a liquid, tablet, capsule or the like.

8. The composition for use according to claim 7, wherein the composition is in a form suitable for oral administration.

9. The composition for use according to claim 7, wherein the composition is in a form suitable for topical administration.

10. The composition for use according to claim 9, which is administered in the form of a cream, ointment, or liquid.

11. The composition according to any one of claims 1 to 10, wherein the animal or human is immunosuppressed or immunocompromised.

## Patentansprüche

1. Zusammensetzung, die eine Kombination von
a) einer Minimalkonzentration von 0,5% (w/v) an von Kohlenhydrat abgeleiteter Fulvinsäure (CHD-FA) oder ein Salz davon und
b) Fluconazol
umfasst, zur Verwendung in einem Verfahren zur Behandlung einer Pilzinfektion eines menschlichen oder nichtmenschlichen tierischen Körpers, wobei das Verfahren umfasst, dass man eine therapeutisch wirksame Menge der Zusammensetzung einem Individuum, das ihrer bedarf, verabreicht, wobei die Pilzinfektion durch einen arzneistoffresistenten Pilz verursacht wird und wobei es sich bei der Pilzinfektion um eine Pilzinfektion mit *Candida albicans, Candida tropicalis, Candida parapsilosis* oder *Candida krusei* handelt.

2. Zusammensetzung, die eine Kombination von
a) einer Minimalkonzentration von 1% (w/v) an CHD-FA und
b) Fluconazol
umfasst, zur Verwendung in einem Verfahren zur Behandlung einer Pilzinfektion eines menschlichen oder nichtmenschlichen tierischen Körpers, wobei das Verfahren umfasst, dass man eine therapeutisch wirksame Menge der Zusammensetzung einem Individuum, das ihrer bedarf, verabreicht, wobei die Pilzinfektion durch einen arzneistoffresistenten Pilz verursacht wird und wobei es sich bei der Pilzinfektion um eine Pilzinfektion mit *Candida glabrata* handelt.

3. Zusammensetzung, die eine Kombination von
a) einer Minimalkonzentration von 0,25% (w/v) an CHD-FA und
b) Amphotericin B
umfasst, zur Verwendung in einem Verfahren zur Behandlung einer Pilzinfektion eines menschlichen oder nichtmenschlichen tierischen Körpers, wobei das Verfahren umfasst, dass man eine therapeutisch wirksame Menge der Zusammensetzung einem Individuum, das ihrer bedarf, verabreicht, wobei die Pilzinfektion durch einen arzneistoffresistenten Pilz verursacht wird und wobei es sich bei der Pilzinfektion um eine Pilzinfektion mit *Aspergillus* handelt.

4. Zusammensetzung zur Verwendung nach einem der Ansprüche 1 bis 3, wobei das Salz das Natrium- oder Kaliumsalz umfasst.

5. Zusammensetzung zur Verwendung nach Anspruch 1 oder Anspruch 4 wie von Anspruch 1 abhängig, die ungefähr 10 ml/kg einer Lösung von ungefähr 0,25% bis ungefähr 1% (w/v) Fulvinsäure oder einem Salz davon und ungefähr 10 mg/kg Fluconazol umfasst.

6. Zusammensetzung zur Verwendung nach Anspruch 3 oder Anspruch 4 wie von Anspruch 3 abhängig, die eine Lösung von ungefähr 0,25% (w/v) Fulvinsäure oder einem Salz davon und ungefähr 0,06 mg/l bis ungefähr 0,5 mg/l Amphotericin B umfasst.

7. Zusammensetzung zur Verwendung nach einem der Ansprüche 1 bis 6, die in Form einer Flüssigkeit, einer Tablette, einer Kapsel oder dergleichen verabreicht wird.

8. Zusammensetzung zur Verwendung nach Anspruch 7, wobei die Zusammensetzung in einer Form, die sich für die orale Verabreichung eignet, vorliegt.

9. Zusammensetzung zur Verwendung nach Anspruch 7, wobei die Zusammensetzung in einer Form, die sich für die topische Verabreichung eignet, vorliegt.

10. Zusammensetzung zur Verwendung nach Anspruch 9, die in Form einer Creme, einer Salbe oder einer Flüssigkeit verabreicht wird.

11. Zusammensetzung nach einem der Ansprüche 1 bis 10, wobei das Tier oder der Mensch immunsupprimiert oder immunkompromitiert ist.

## Revendications

1. Composition comprenant une combinaison de :
a) une concentration minimale de 0,5 % (p/v) d'acide fulvique dérivé d'un glucide (CHD-FA) ou d'un sel de celui-ci, et
b) fluconazole,
pour son utilisation dans un procédé de traitement d'une infection fongique de l'organisme d'un être humain ou d'un animal non humain, le procédé comprenant l'administration à un sujet qui le nécessite d'une quantité thérapeutiquement efficace de la composition, l'infection fongique étant causée par un champignon pharmacorésistant, et l'infection fongique étant une infection fongique à *Candida albicans, Candida tropicalis, Candida parapsilosis* ou *Candida krusei.*

2. Composition comprenant une combinaison de :
a) une concentration minimale de 1 % (p/v) de CHD-FA, et
b) fluconazole,
pour son utilisation dans un procédé de traitement d'une infection fongique de l'organisme d'un être humain ou d'un animal non humain, le procédé comprenant l'administration à un sujet qui le nécessite d'une quantité thérapeutiquement efficace de la composition, l'infection fongique étant causée par un champignon pharmacorésistant, et l'infection fongique étant une infection fongique à *Candida glabrata.*

3. Composition comprenant une combinaison de :
a) une concentration minimale de 0,25 % (p/v) de CHD-FA, et
b) amphotéricine B,
pour son utilisation dans un procédé de traitement d'une infection fongique de l'organisme d'un être humain ou d'un animal non humain, le procédé comprenant l'administration à un sujet qui le nécessite d'une quantité thérapeutiquement efficace de la composition, l'infection fongique étant causée par un champignon pharmacorésistant, et l'infection fongique étant une infection fongique à *Aspergillus.*

4. Composition pour son utilisation selon l'une quelconque des revendications 1 à 3, dans laquelle le sel comprend le sel de sodium ou de potassium.

5. Composition pour son utilisation selon la revendication 1 ou la revendication 4 lorsqu'elle dépend de la revendication 1, comprenant environ 10 ml/kg d'une solution d'environ 0,25 % à environ 1 % (p/v) d'acide fulvique, ou d'un sel de celui-ci, et environ 10 mg/kg de fluconazole.

6. Composition pour son utilisation selon la revendication 3 ou la revendication 4 lorsqu'elle dépend de la revendication 3, comprenant une solution d'environ 0,25 % (p/v) d'acide fulvique, ou d'un sel de celui-ci, et environ 0,06 mg/l à environ 0,5 mg/l d'amphotéricine B.

7. Composition pour son utilisation selon l'une quelconque des revendications 1 à 6 qui est administrée sous la forme d'un liquide, d'un comprimé, d'une capsule ou autre.

8. Composition pour son utilisation selon la revendication 7, laquelle composition se présente sous une forme adaptée à l'administration orale.

9. Composition pour son utilisation selon la revendication 7, laquelle composition se présente sous une forme adaptée à l'administration topique.

10. Composition pour son utilisation selon la revendication 9, qui est administrée sous la forme d'une crème, d'un onguent ou d'un liquide.

11. Composition selon l'une quelconque des revendications 1 à 10, dans laquelle l'animal ou l'être humain est immunodéprimé ou immunovulnérable.
